# EUROPEAN PATENT SPECIFICATION

(11) **EP 3 954 333 B1**
(45) Date of publication and mention of the grant of the patent: **03.05.2023**
(21) Application number: 21190091.5
(22) Date of filing: 06.08.2021
(51) Int. Cl.: A61F 2/32, A61F 2/36

(54) **HIP PROSTHESIS WITH IMPROVED STEM**
HÜFTPROTHESE MIT VERBESSERTEM SCHAFT
PROTHÈSE DE HANCHE PRÉSENTANT UNE MEILLEURE TIGE

(30) Priority: 13.08.2020 IT 202000020116
(43) Date of publication of application: 16.02.2022
(73) Proprietor: Melozzi, Alessandro, 64100 Teramo (TE) (IT)
(72) Inventor: Melozzi, Alessandro, 64100 Teramo (TE) (IT)
(74) Representative: Cutropia, Gianluigi

(56) References cited:
- EP-A1- 0 528 284
- EP-A1- 0 865 777
- EP-A1- 1 072 237
- EP-A2- 0 721 767
- DE-A1- 19 610 729
- FR-A1- 2 959 659
- US-A- 5 888 210

## Description

The present invention relates to a hip prosthesis having a stem with a suitable structure for adjusting to the configuration of the femoral channel.

As it is known, a hip prosthesis is used to reconstruct the head of a femur of a patient. Therefore, such a hip prosthesis comprises a stem suitable for being disposed in the femoral channel, and a head fixed to the stem and suitable for being engaged in an acetabular seat of the patient. Generally, the stem has a truncated conical shape that is symmetrical relative to an axis, with decreasing dimensions going from up downwards.

Long-stemmed prostheses, wherein the stem of the prosthesis penetrates deeply in the femoral channel, and short-stemmed prostheses, wherein the stem of the prosthesis penetrates in the femoral channel for a shorter length compared to a long stem, are known.

In comparison with long-stemmed prostheses, short-stemmed prostheses are less invasive, are easier to be inserted in the femoral channel and are capable of reducing the amount of bone to be removed. However, short-stemmed prostheses are impaired by drawbacks related with stability and durability.

For these reasons, long-stemmed prostheses are still preferred although they are impaired by the fact that they cannot adapt to the shape of the femoral channel.

Two models of prostheses are currently available on the market, namely prostheses wherein the stem is fixed to the femur with cement and prosthesis wherein the stem is fixed to the femur without using cement.

The prostheses that do not use cement have a low reliability because the stem is not firmly anchored to the femur.

On the other hand, the prostheses that use cement are impaired by the fact that they are very invasive because the cement can invade the femoral channel.

EP0528284A1 discloses a hip joint prosthesis provided with a helically twisted shaft with rectangular section.

EP1072237A1 discloses a hip endoprosthesis provided with a shaft having an upper curved portion and a lower straight portion.

EP0721767A2 discloses a hip endoprosthesis provided with a shaft that is especially designed for cementless anchoring. Said shaft is composed of a profile with E-shaped section in such a way to define grooves for anchoring the bony sponge. The shaft is provided with opening or recesses disposed in an upper wall of the shaft for anchoring the bony sponge. Said openings are not shaped like a pocket, do not have a channel for injecting the cement and are not suitable for receiving and holding the cement.

The purpose of the present invention is to eliminate the drawbacks of the prior art by disclosing a hip prosthesis provided with a stem suitable for adjusting to the configuration of the femoral channel wherein it is installed.

Another purpose of the present invention is to disclose a hip prosthesis that is safe, reliable and easy to install for the surgeon.

These purposes are achieved according to the invention with the characteristics of the independent claim 1.

Advantageous embodiments of the invention appear from the dependent claims.

Additional features of the invention will appear clear from the detailed description below, which refers to merely illustrative, not limiting embodiments, shown in the appended drawings, wherein:
Fig. 1 is a view of a prosthesis according to the invention, wherein the stem is disposed in a femoral channel shown in a sectional view and the head is shown in a perspective view and is separated from the stem;
Fig. 2 is a side view of a head of a femur before the application of the prosthesis according to the invention.
Fig. 3 is an axial view of a portion of a femur prepared for accommodating the prosthesis according to the invention;
Fig. 4 is a side view of the stem of the hip prosthesis according to the invention;
Fig. 4A is a side view of the stem of Fig. 4, which shows the radiuses of curvature of the upper portion of the stem;
Fig. 5 is the same view as Fig. 4A, which shows three sectional planes of the stem;
Fig. 6 shows three sections of the stem, taken along the planes I-I, II-II and III-III of Fig. 5.
Fig. 7 are views of six portions of the stem, taken along the sectional planes of Fig. 4;
Fig. 8 is a back view of the stem of Fig. 4;
Fig. 9 is a back view of the stem of Fig. 4;
Fig. 10 is a perspective view of an upper portion of the stem; and
Fig. 11 is a sectional view of the upper portion of the stem, taken along the sectional plane XI-XI of Fig. 10.

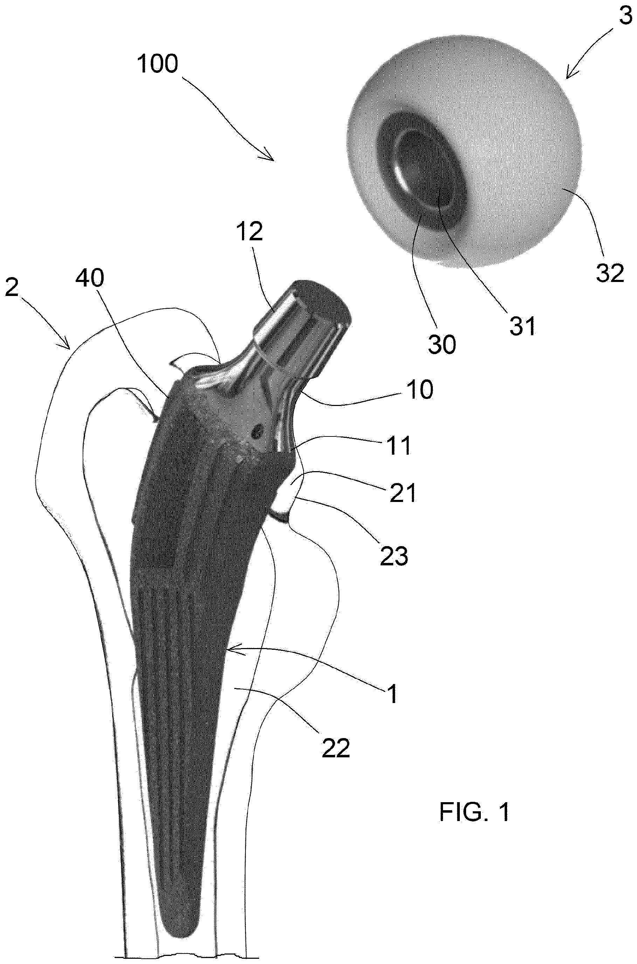

Fig. 1 illustrates a hip prosthesis according to the invention, which is generally indicated with reference numeral 100.

The prosthesis (100) comprises:
- a stem (1) suitable for being inserted in a channel (22) of a femur (2) of a patient, and
- a head assembly (3) suitable for being fixed to the stem (1) and disposed in an acetabular seat of the patient.

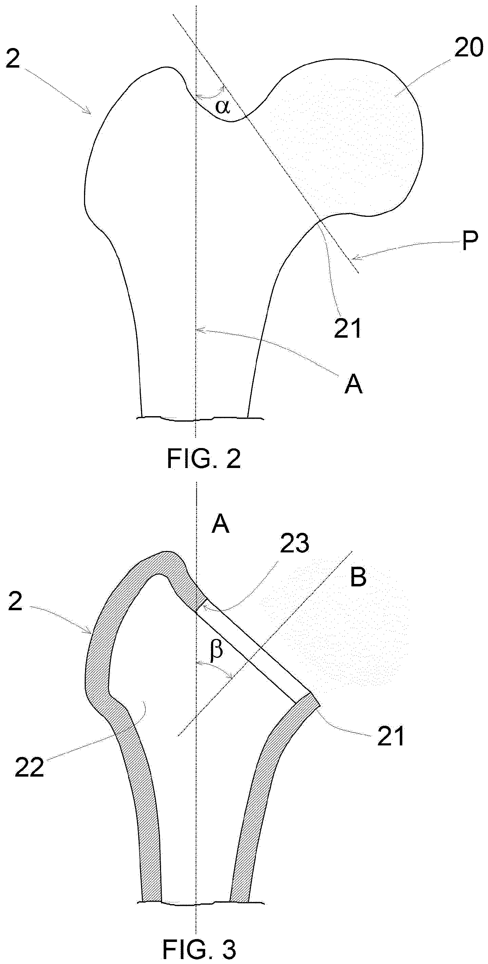

With reference to Figs. 2 and 3, the femur (2) has a head (20), a neck (21) and a channel (22) that extends axially in the femur. The channel (22) of the femur has an axis (A).

In order to prepare the femur (2) to accommodate the prosthesis (100), the head (20) of the femur is removed by cutting the bone of the head along a plane (P) inclined with respect to the axis (A) of the femoral channel by an angle (α) of approximately 30-50°. A hole (23) is obtained in the neck (21) of the femur by means of a surgical rasp in order to access the channel (22). The hole (23) has an axis (B) inclined by an angle (β) of approximately 30-50° with respect to the axis (A) of the channel. The hole (23) of the femoral neck has a truncated conical shape with a decreasing diameter going toward the channel and with a coning angle of approximately 1-3°.

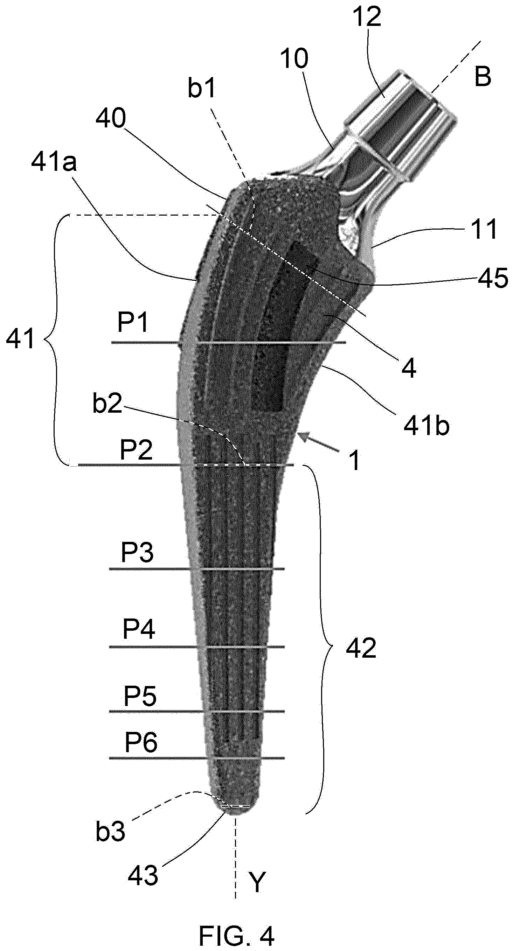

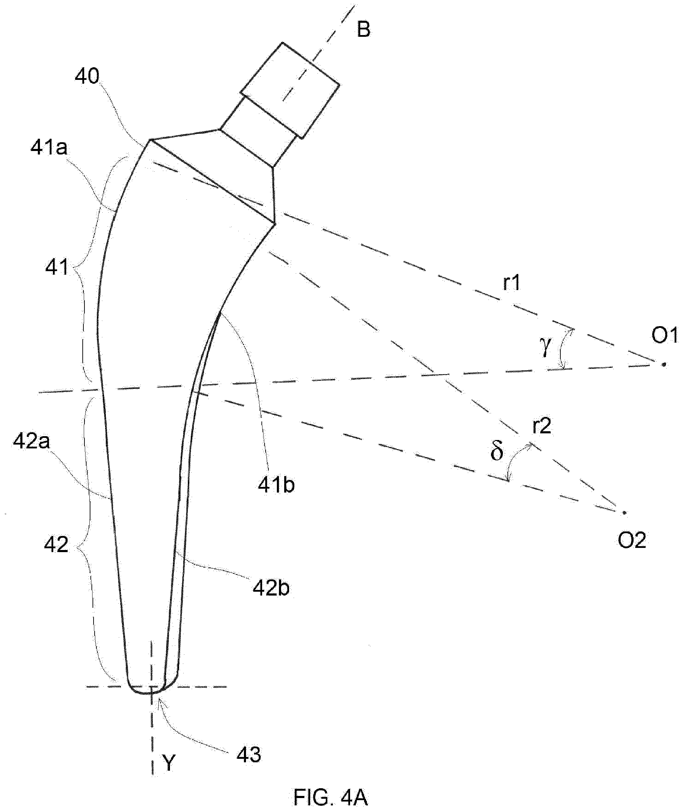

With reference to Figs. 1 and 4, the stem (1) has a body (4) having a coupling portion (40) suitable for being coupled in pressure-fit coupling mode with the hole (23) of the femoral neck. For such a purpose, the coupling portion (40) has a tapered or truncated conical shape with a coning angle of about 1-3°.

The coupling portion (40) of the body of the stem continues with an upper portion (41) with curved tapered shape, joined to a lower portion (42) with tapered shape, ending with a point (43) with rounded beveled shape in order to avoid any overpressure on the cortical.

The upper portion (41) and the lower portion (42) are suitable for being disposed in the channel (22) of the femur.

The upper portion (41) and the lower portion (42) have decreasing dimensions going downwards. The length of the upper portion (41) is approximately 50-60% of the length of the lower portion (42).

The upper portion (41) and the lower portion (42) have a rectangular or elliptical cross-section having a major axis and a minor axis and therefore two major faces and two minor faces.

The upper portion (41) has a convex surface (41a) suitable for being directed upwards inside the femoral channel, and a concave surface (41b) suitable for being directed downwards inside the femoral channel.

With reference to Fig. 4A, the convex surface (41a) of the upper portion has a profile shaped like an arc of circle with center (O1), radius of curvature (r1) and is subtended by an angle (γ) of approximately 20-30°.

The concave surface (41b) of the upper portion has a profile shaped like an arc of circle with center (02), radius of curvature (r2) and is subtended by an angle (δ) of approximately 20-30°.

The center (O1) of the profile of the convex surface (41a) is above the center (02) of the profile of the concave surface and the radius of curvature (r1) of the convex surface is higher than the radius of curvature (r2) of the concave surface.

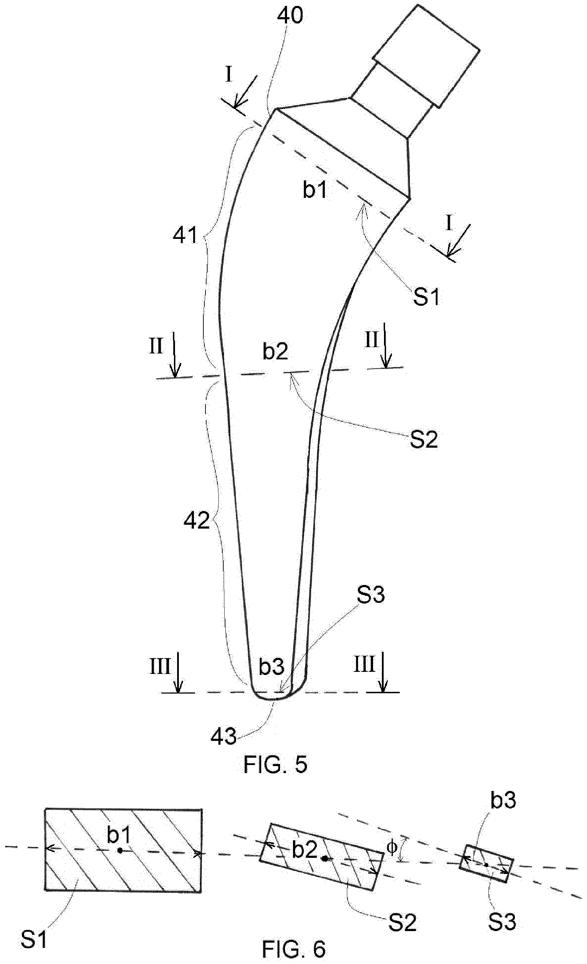

With reference to Figs. 5 and 6, a transverse section (S1) with a first major transverse axis (b1) is provided between the coupling portion (40) and the upper portion (41).

A transverse section (S2) with a second major transverse axis (b2) is provided between the upper portion (41) and the lower portion (42). The length of the second major transverse axis (b2) is approximately 50-70% of the length of the first major transverse axis (b1).

The axis (Y) of the lower portion (42) is suitable for coinciding with the axis (A) of the femoral channel. A transverse section (S3) with a third major transverse axis (b3) is provided between the lower portion (42) and the point (43). The length of the third major transverse axis (b3) is approximately 40-60% of the length of the second major transverse axis (b2).

The lower portion (42) has a tapered shape with lateral profiles (42a, 42b) (Fig. 4A) having a tapering angle of 2-10°.

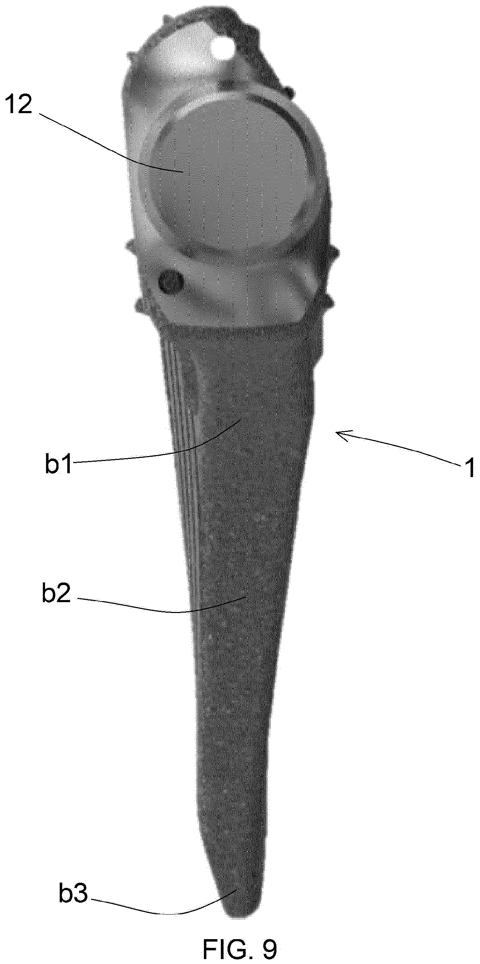

The upper portion (41) and the lower portion (42) are helically twisted in such a way that the third major transverse axis (b3) of the transverse section (S3) between the lower portion (42) and the point (43) is rotated by a torsional angle (φ) (Fig. 9) of 10-30° relative to the first major transverse axis (b1) of the transverse section (S1) between the coupling portion (40) and the upper portion (41).

In Fig. 4, the body (4) of the stem is cut along six horizontal planes (P1 - P6).

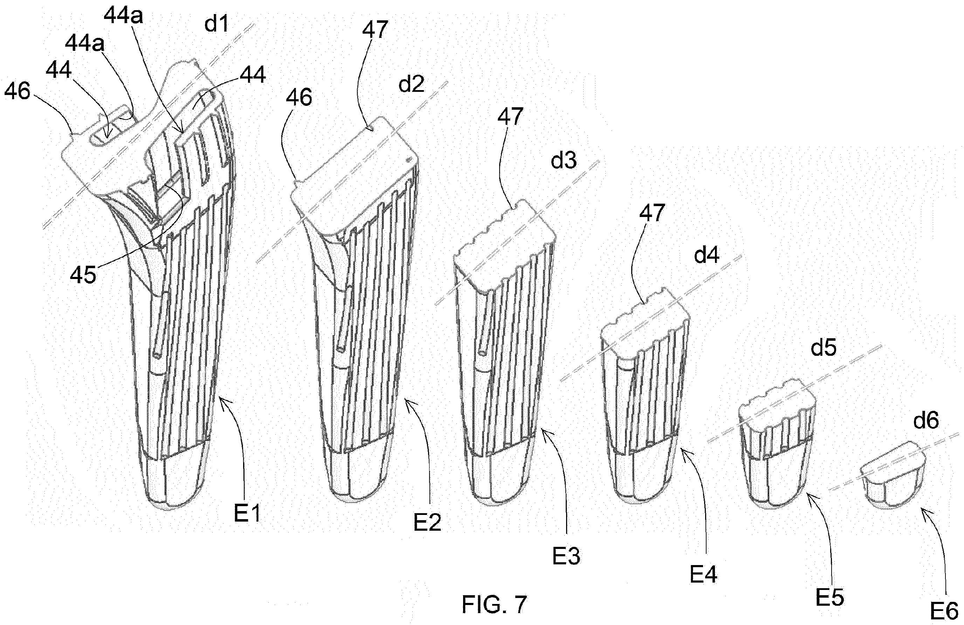

Fig. 7 shows six portions (E1- E6) of the body of the stem cut along the horizontal planes (P1-P6) of Fig. 4.

Each portion (E1-E6) has a section with a respective major transverse axis (d1- d6) that extends along the major dimension of the section. As shown in Fig. 4, the major transverse axes (d2-d6) of the portions (P2-P6) following the first portion (P1) are rotated gradually by an increasing angle relative to the major transverse axis (d1) of the first portion.

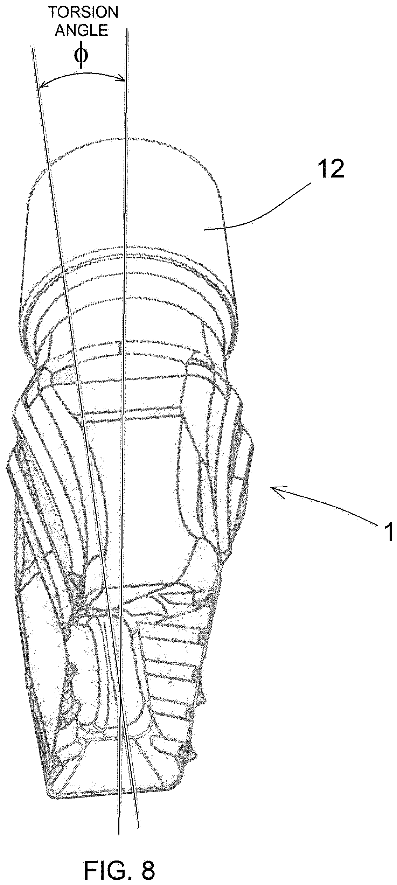

Figs. 8 and 9 show the torsional angle (φ) between the first major transverse axis (b1) of the transverse section (S1) between the coupling portion (40) and the upper portion (41) and the third major transverse axis (b3) of the transverse section (S2) between the lower portion (42) and the point (43). Due to such a twisted shape of the body of the stem, the stem can be easily inserted in the femoral channel (22) by rotating the stem in a screwing direction.

The body (4) of the stem has a plurality of ribs (46) and/or grooves (47) that extend longitudinally in the major sides of the upper portion (41) and of the lower portion (42).

Advantageously, the upper portion (41) is provided with ribs (46) and the lower portion (42) is provided with grooves (47).

The stem (1) has a collar (10) joined to the coupling portion (40) by means of a shoulder (11). A shank (12) is disposed on the collar (10). The shoulder (11), the collar (10) and the shank (12) are suitable for being disposed outside the femoral channel.

With reference to Fig. 1, the shank (12) has a truncated conical shape with decreasing diameter going upwards for a Morse taper coupling with the head assembly (3). The head assembly (3) has a head (30) with spherical shape provided with a hole (31) with truncated conical shape for coupling with the shank (12) of the stem. The coning angles of the shank (12) and of the hole (31) of the head are approximately 1-3°.

A cap (31) is mounted on the head (30) and is suitable for being fixed in an acetabular seat of the patient.

The shank (12) has an axis (C) that corresponds to the axis of the coupling portion (40) of the body of the stem. When the body of the stem is installed in the femur, the axis (C) of the shank coincides with the axis (B) of the hole (23) of the neck of the femur and is therefore inclined by the angle (β) relative to the axis (A) of the femoral channel. Therefore, the axis (C) of the shank (12) is inclined relative to the axis (Y) of the lower portion (42) by the angle (β) of 30-50°.

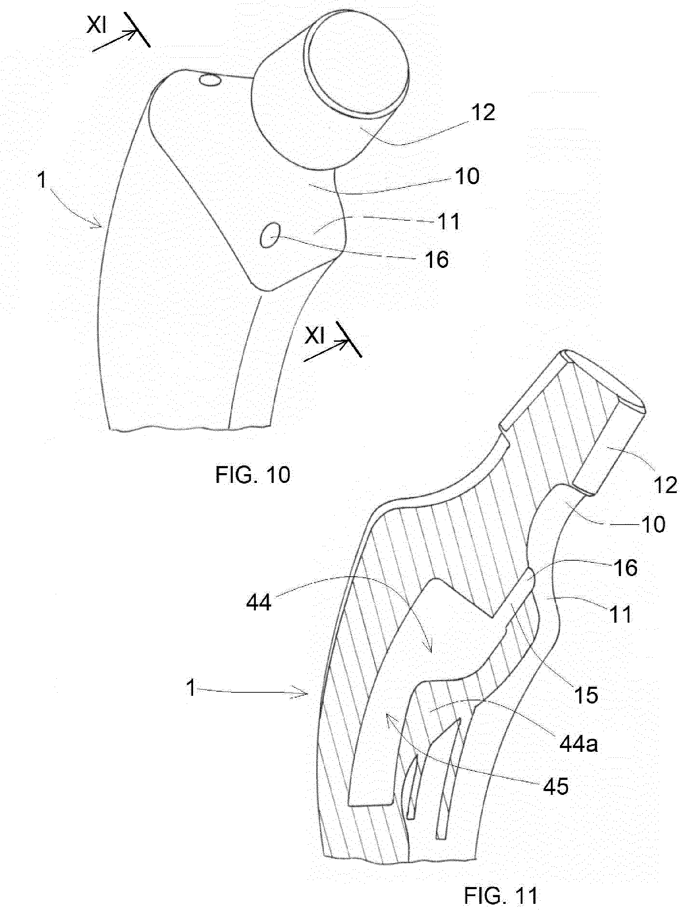

With reference to Figs. 7, 10 and 11, the body (4) of the stem has two chambers (44) shaped like a pocket and disposed in the curved tapered portion of the stem, in opposite positions relative to a major transverse axis. Each chamber (44) can be accessed by means of an injection channel (15) provided with an inlet hole (16) in the shoulder (11) of the stem for the injection of material from outside.

The injected material can be acrylic cement to improve the anchoring of the stem, or synthetic bone with the addition of antibiotics to prevent injections or platelet gel to stimulate bony growth.

The injection channel (15) is substantially parallel to the axis of the stem. It must be noted that, when the stem is fixed to the neck of the femur, the shoulder (11) of the stem remains outside the femur and material can be injected through the inlet hole (16).

The chamber (44) communicates with an outlet slot (45) obtained on the surface of the upper portion (41) of the stem for the coming out of the injected material that fills the chamber (44). In view of the above, the material comes out of the chamber (44) and goes in contact with the bone of the femur inside the femoral channel.

In a cross-sectional view, the outlet slot (45) and the chamber (44) are shaped like an L rotated by 90°, in such a way that the chamber (44) has an undercut wall (44a) suitable for holding the injected material.

The chambers (44) are used to hold the cement or another anchoring material used for anchoring the stem to the bone of the femur. In such a way, the cement enters the injection channel (15), penetrates in the chamber (44), fills the chamber (44) and comes out of the outlet slot (45) to go in contact with the bone of the femur. It must be noted that the cement remains partially in the chamber (44) and is held by the undercut wall (44a) of the chamber (44).

The body (4) of the stem can be made of titanium and can be coated with trabecular titanium or porous titanium in the upper portion (41) and in the lower portion (42).

It must be considered that two different models of prostheses are currently available on the market, namely models wherein the stem is fixed to the femur with cement and models wherein the stem is fixed to the femur without cement.

The advantage of the stem (1) of the prosthesis according to the invention is that it can be fixed in the femoral channel either without cement or with cement. With such a solution, it is possible to use only this type of stem with the aforementioned advantages.

Additionally, the cavities (44) that are shaped like a pocket can be filled with cement, preventing the cement from completely invading the femoral channel, with significant advantages also in case of revision of the hip prosthesis.

## Claims

1. Hip prosthesis (100) comprising:
- a stem (1) comprising a body (4) with a coupling portion (40) that continues with an upper portion (41) and a lower portion (42) ending with a point (43), wherein the stem (1) comprises a collar (10) connected to the coupling portion (40) by means of a shoulder (11) and a shank (12) disposed on the collar (10), wherein the shoulder (11), the collar (10) and the shank (12) are suitable for being disposed outside the femoral channel, and
- a head assembly (3) suitable for being fixed to the stem (1) and
wherein said coupling portion (40) is suitable for being disposed in correspondence of a hole (23) of a neck (21) of a femur of a patient; said upper portion (41) and lower portion (42) and point (43) are suitable for being inserted in a channel (22) of the femur (2) and said head assembly (3) is suitable for being disposed in an acetabular seat of the patient;
wherein said upper portion (41) has a curved tapered shape and said lower portion (42) has a tapered shape,
wherein said upper portion (41) and said lower portion (42) have a rectangular or elliptical cross-section with a major axis and a minor axis;
wherein a transverse section (S1) with a first major transverse axis (b1) is disposed between the coupling portion (40) and the upper portion (41); a transverse section (S2) with a second major transverse axis (b2) is disposed between the upper portion (41) and the lower portion (42); and a transverse section (S3) with a third major transverse axis (b3) is disposed between the lower portion (42) and the point (43);
wherein the upper portion (41) and the lower portion (42) are helically twisted, in such a way that the third major transverse axis (b3) of the transverse section (S3) between the lower portion (42) and the point (43) is rotated by a torsional angle (φ) of 10-30° relative to the first major transverse axis (b1) of the transverse section (S1) between the coupling portion (40) and the upper portion (41);
**characterized in that**
the body (4) of the stem has two chambers (44) shaped like a pocket and disposed in the upper portion (41) of the body of the stem in opposite positions relative to a major transverse axis; each chamber (44) being accessed by means of an injection channel (15) provided with an inlet hole (16) in the shoulder (11) of the stem; and
the chamber (44) communicates with an outlet slot (45) obtained on the surface of the upper portion (41) of the body of the stem for the coming out of the injected material that fills the chamber (44).

2. The prosthesis (100) of claim 1, wherein, in a cross-sectional view, the outlet slot (45) and the chamber (44), are shaped like an L rotated by 90° in such a way that the chamber (44) has an undercut wall (44a) suitable for holding the material injected in the chamber (44).

3. The prosthesis (100) of claim 1 or 2, wherein the upper portion (41) has a convex surface (41a) suitable for being directed upwards inside the femoral channel, and a concave surface (41b) suitable for being directed downwards inside the femoral channel;
the convex surface (41a) of the upper portion has a profile shaped like an arc of circle with center (O1), radius of curvature (r1) and is subtended by an angle (γ) of approximately 20-30°; the concave surface (41b) of the upper portion has a profile shaped like an arc of circle with center (02), radius of curvature (r2) and is subtended by an angle (δ) of approximately 20-30°;
the center (O1) of the profile of the convex surface (41a) is above the center (02) of the profile of the concave surface and the radius of curvature (r1) of the convex surface is higher than the radius of curvature (r2) of the concave surface.

4. The prosthesis (100) according to any one of the preceding claims, wherein the length of the upper portion (41) is approximately 80-90% of the length of the lower portion (42).

5. The prosthesis (100) according to any one of the preceding claims, wherein the length of the second major transverse axis (b2) of the transverse section between the upper portion (41) and the lower portion (42) is approximately 50-70% of the length of the first major transverse axis (b1) of the transverse section between the coupling portion (40) and the upper portion (41).

6. The prosthesis (100) according to any one of the preceding claims, wherein the length of the third major transverse axis (b3) of the transverse section (S3) between the lower portion (42) and the point (43) is approximately 40-60% of the length of the second major transverse axis (b2) of the transverse section (S3) between the upper portion (41) and the lower portion (42).

7. The prosthesis (100) of any one of the preceding claims, wherein the lower portion (42) has an axis (Y) suitable for coinciding with the axis (A) of the femoral channel and has a tapered shape with lateral profiles (42a, 42b) having a tapering angle of 2-10°.

8. The prosthesis (100) of claim 7, wherein said shank (12) has a truncated conical shape with decreasing diameter going upwards for a Morse taper coupling with the head assembly (3);
the shank (12) has an axis (C) inclined relative to the axis (Y) of the lower portion (42) by an angle (β) of 30-50°.

9. The prosthesis (100) according to any one of the preceding claims, wherein the body (4) of the stem has a plurality of ribs (46) and/or grooves (47) that extend longitudinally in the major sides of the upper portion (41) and of the lower portion (42).

## Patentansprüche

1. Hüftprothese (100), umfassend:
- einen Schaft (1), umfassend einen Körper (4) mit einem Kopplungsabschnitt (40), der sich in einem oberen Abschnitt (41) fortsetzt, und mit einem unteren Abschnitt (42), der in einer Spitze (43) endet, wobei der Schaft (1) einen Hals (10), der mit dem Kopplungsabschnitt (40) mittels einer Schulter (11) verbunden ist, und einen Zapfen (12), der am Hals (10) angeordnet ist, umfasst, wobei die Schulter (11), der Hals (10) und der Zapfen (12) dazu bestimmt sind, außerhalb des Oberschenkelknochenkanals angeordnet zu werden, und
- eine Kopfanordnung (3), die geeignet ist, an dem Schaft (1) befestigt zu werden, und
wobei der Kopplungsabschnitt (40) geeignet ist, an einem Loch (23) eines Halses (21) eines Oberschenkelknochens eines Patienten angeordnet zu werden; wobei der obere Abschnitt (41) und der untere Abschnitt (42) und die Spitze (43) geeignet sind, in einen Kanal (22) des Oberschenkelknochens (2) eingebracht zu werden, und wobei die Kopfanordnung (3) geeignet ist, in einem Pfannensitz des Patienten angeordnet zu werden;
wobei der obere Abschnitt (41) eine gebogene und spitz zulaufende Form aufweist und der untere Abschnitt (42) eine spitz zulaufende Form aufweist,
wobei der obere Abschnitt (41) und der untere Abschnitt (42) einen rechteckigen Querschnitt oder einen elliptischen Querschnitt mit einer Hauptachse und einer Nebenachse aufweisen;
wobei zwischen dem Kopplungsabschnitt (40) und dem oberen Abschnitt (41) sich ein Querabschnitt (S1) mit einer ersten Hauptquerachse (b1) befindet; zwischen dem oberen Abschnitt (41) und dem unteren Abschnitt (42) sich ein Querabschnitt (S2) mit einer zweiten Hauptquerachse (b2) befindet; und zwischen dem unteren Abschnitt (42) und der Spitze (43) sich ein Querabschnitt (S3) mit einer dritten Hauptquerachse (b3) befindet;
wobei der obere Abschnitt (41) und der untere Abschnitt (42) schraubenförmig verdrillt sind, so dass die dritte Hauptquerachse (b3) des Querabschnitts (S3) zwischen dem unteren Abschnitt (42) und der Spitze (43) um einen Verdrehwinkel (φ) von 10 - 30° in Bezug auf die erste Hauptquerachse (b1) des Querabschnitts (S1) zwischen Kopplungsabschnitt (40) und dem oberen Abschnitt (41) gedreht ist;
**dadurch gekennzeichnet, dass**
der Körper (4) des Schaftes zwei taschenartige Kammern (44) aufweist, die im oberen Abschnitt (41) des Körpers des Schaftes in gegenüberliegenden Positionen in Bezug auf eine Hauptquerachse angeordnet sind; wobei jede Kammer (44) über einen Injektionskanal (15) mit einem Eingangsloch (16) in der Schulter (11) des Schaftes zugänglich ist; und
die Kammer (44) mit einem Auslaufschlitz (45) in Verbindung steht, der auf der Oberfläche des oberen Abschnitts (41) des Schaftes zum Auslaufen des injizierten Materials herausgearbeitet ist, mit dem die Kammer (44) gefüllt ist.

2. Prothese (100) nach Anspruch 1, wobei der Auslaufschlitz (45) und die Kammer (44) in der Querschnittansicht die Form eines um 90° gedrehten "L" aufweisen, derart, dass die Kammer (44) eine hinterschnittene Wand (44a) aufweist, die geeignet ist, das in die Kammer injizierte Material zu halten.

3. Prothese (100) nach Anspruch 1 oder 2, wobei der obere Abschnitt (41) eine konvexe Oberfläche (41a) aufweist, die dazu bestimmt ist, in dem Oberschenkelknochenkanal nach oben gerichtet zu werden, und eine konkave Oberfläche (41b), die dazu bestimmt ist, in dem Oberschenkelknochenkanal nach unten gerichtet zu werden;
wobei die konvexe Oberfläche (41a) des oberen Abschnitts ein kreisbogenförmiges Profil mit dem Mittelpunkt (O1), Krümmungsradius (r1) und einem Winkel (γ) von circa 20 - 30° aufweist; wobei die konkave Oberfläche (41b) des oberen Abschnitts ein kreisbogenförmiges Profil mit dem Mittelpunkt (02), Krümmungsradius (r2) und einem Winkel (δ) von circa 20 - 30° aufweist;
der Mittelpunkt (O1) des Profils der konvexen Oberfläche (41a) sich über dem Mittelpunkt (02) des Profils der konkaven Oberfläche befindet und der Krümmungsradius (r1) der konvexen Oberfläche größer als der Krümmungsradius (r2) der konkaven Oberfläche ist.

4. Prothese (100) nach einem der vorstehenden Ansprüche, wobei die Länge des oberen Abschnitts (41) circa 80 - 90 % der Länge des unteren Abschnitts (42) beträgt.

5. Prothese (100) nach einem der vorstehenden Ansprüche, wobei die zweite Hauptquerachse (b2) des Querabschnitts (S2) zwischen dem oberen Abschnitt (41) und dem unteren Abschnitt (42) eine Länge von circa 50 - 70 % der Länge der ersten Hauptquerachse (b1) des Querabschnitts (S1) zwischen dem Kopplungsabschnitt (40) und dem oberen Abschnitt (41) aufweist.

6. Prothese (100) nach einem der vorstehenden Ansprüche, wobei die dritte Hauptquerachse (b3) des Querabschnitts (S3) zwischen dem unteren Abschnitt (42) und der Spitze (43) eine Länge von circa 40 - 60 % der zweiten Hauptquerachse (b2) des Querabschnitts (S3) zwischen dem oberen Abschnitt (41) und dem unteren Abschnitt (42) aufweist.

7. Prothese (100) nach einem der vorstehenden Ansprüche, wobei der untere Abschnitt (42) eine Achse (Y) aufweist, die dazu bestimmt ist, mit der Achse (A) des Oberschenkelknochenkanals zusammenzufallen und eine spitz zulaufende Form mit seitlichen Profilen (42a, 42b) aufweist, die einen Verjüngungswinkel von 2 - 10° aufweisen.

8. Prothese (100) nach Anspruch 7, wobei der Zapfen (12) des Schaftes die Form eines Kegelstumpfes mit einem nach oben abnehmenden Durchmesser für eine Morsekonuskopplung mit der Kopfanordnung (3) aufweist;
wobei der Zapfen (12) eine Achse (C) aufweist, die in Bezug auf die Achse (Y) des unteren Abschnitts (42) um einen Winkel (β) von 30 - 50° geneigt ist.

9. Prothese (100) nach einem der vorstehenden Ansprüche, wobei der Körper (4) des Schaftes eine Mehrzahl von Rippen (46) und/oder Rillen (47) aufweist, die sich auf den Hauptseiten des oberen Abschnitts (41) und des unteren Abschnitts (42) längs erstrecken.

## Revendications

1. Prothèse (100) de hanche comprenant :
- une tige (1) comprenant un corps (4) ayant une portion d'accouplement (40) qui continue avec une portion supérieure (41) et une portion inférieure (42) terminant en une pointe (43), où la tige (1) comprend un collier (10) relié à la portion d'accouplement (40) moyennant un épaulement (11) et une queue (12) disposée sur le collier (10), où l'épaulement (11), le collier (10) et la queue (12) sont destinés à être disposés à l'extérieur du canal du fémur, et
- un ensemble tête (3) apte à être fixé à la tige (1) et
où ladite portion d'accouplement (40) est apte à être disposée en correspondance d'un orifice (23) du col (21) du fémur d'un patient; lesdites portion supérieure (41) et portion inférieure (42) et pointe (43) sont aptes à être insérées dans un canal (22) du fémur (2) et ledit ensemble tête (3) est apte à être disposé dans un logement acétabulaire du patient ;
où ladite portion supérieure (41) a une forme courbe et conique et ladite portion inférieure (42) a une forme conique,
où ladite portion supérieure (41) et ladite portion inférieure (42) ont une section transversale rectangulaire ou elliptique avec un axe majeur et un axe mineur ;
où entre la portion d'accouplement (40) et la portion supérieure (41) il y a une section transversale (S1) ayant un premier axe majeur transversal (b1) ; entre la portion supérieure (41) et la portion inférieure (42) il y a une section transversale (S2) ayant un deuxième axe majeur transversal (b2) ; et entre la portion inférieure (42) et la pointe (43) il y a une section transversale (S3) ayant un troisième axe majeur transversal (b3) ;
où la portion supérieure (41) et la portion inférieure (42) sont torsadées hélicoïdalement, de manière que le troisième axe majeur transversal (b3) de la section transversale (S3), entre la portion inférieure (42) et la pointe (43), soit pivoté d'un angle de torsion (φ) de 10-30° par rapport au premier axe majeur transversal (b1) de la section transversale (S1) entre la portion d'accouplement (40) et la portion supérieure (41) ;
**caractérisée en ce que**
le corps (4) de la tige a deux chambres (44), en forme de poches, disposées dans la portion supérieure (41) du corps de la tige en positions opposées par rapport à un axe transversal majeur ; chaque chambre (44) étant accessible moyennant un canal d'injection (15) ayant un orifice d'entrée (16) dans l'épaulement (11) de la tige ; et
la chambre (44) communique avec une fissure de sortie (45) réalisée sur la surface de la portion supérieure (41) du corps de la tige pour la sortie du matériel injecté qui remplit la chambre (44).

2. Prothèse (100) selon la revendication 1, où la fissure de sortie (45) et la chambre (44), vues en section transversale, ont la forme d'un « L » pivoté de 90°, de manière que la chambre (44) ait une contre-dépouille de la paroi (44a) apte à retenir le matériel injecté dans la chambre.

3. Prothèse (100) selon la revendication 1 ou 2, où la portion supérieure (41) a une surface convexe (41a) destinée à être orientée vers le haut dans le canal du fémur, et une surface concave (41b) destinée à être orientée vers le bas dans le canal du fémur ;
la surface convexe (41a) de la portion supérieure a un profil en forme d'arc de cercle avec un centre (O1), un rayon de courbure (r1) et elle est sous-tendue d'un angle (γ) d'environ 20-30°; la surface concave (41b) de la portion supérieure a un profil en forme d'arc de cercle avec un centre (02), un rayon de courbure (r2) et elle est sous-tendue d'un angle (δ) d'environ 20-30° ;
le centre (O1) du profil de la surface convexe (41a) se trouve au-dessus du centre (02) du profil de la surface concave et le rayon de courbure (r1) de la surface convexe est majeur du rayon de courbure (r2) de la surface concave.

4. Prothèse (100) selon l'une quelconque des revendications précédentes, où la longueur de la portion supérieure (41) est d'environ 80-90% de la longueur de la portion inférieure (42).

5. Prothèse (100) selon l'une quelconque des revendications précédentes, où le deuxième axe majeur transversal (b2) de la section transversale (S2), entre la portion supérieure (41) et la portion inférieure (42), a une longueur équivalente à environ le 50-70% de la longueur du premier axe majeur transversal (b1) de la section transversale (S1) entre la portion d'accouplement (40) et la portion supérieure (41).

6. Prothèse (100) selon l'une quelconque des revendications précédentes, où le troisième axe majeur transversal (b3) de la section transversale (S3), entre la portion inférieure (42) et la pointe (43), a une longueur équivalente à environ le 40-60% de la longueur du deuxième axe majeur transversal (b2) de la section transversale (S3) entre la portion supérieure (41) et la portion inférieure (42).

7. Prothèse (100) selon l'une quelconque des revendications précédentes, où la portion inférieure (42) a un axe (Y) destiné à coïncider avec l'axe (A) du canal du fémur et a une forme conique avec des profils latéraux (42a, 42b) ayant un angle de conicité de 2-10°.

8. Prothèse (100) selon la revendication 7, où ladite queue (12) de la tige a une forme tronconique avec diamètre décroissant en allant vers le haut pour un accouplement du type cône Morse avec l'ensemble tête (3) ;
la queue (12) ayant un axe (C) incliné par rapport à l'axe (Y) de la portion inférieure (42) d'un angle (β) de 30-50°.

9. Prothèse (100) selon l'une quelconque des revendications précédentes, où le corps (4) de la tige a une pluralité de nervures (46) et/ou de rainures (47) qui se déploient longitudinalement sur les faces majeures de la portion supérieure (41) et de la portion inférieure (42).
